(19) Europäisches Patentamt
European Patent Office
Office européen des brevets

(11) EP 0 894 490 B1

(12) # FASCICULE DE BREVET EUROPEEN

(45) Date de publication et mention de la délivrance du brevet:
**16.01.2002 Bulletin 2002/03**

(51) Int Cl.[7]: **A61K 7/043**, A61K 7/48, A61K 7/06, A61K 7/032

(21) Numéro de dépôt: **98401143.7**

(22) Date de dépôt: **13.05.1998**

(54) **Nouvelles compositions cosmétiques comprenant un polymère filmogène**

Kosmetische Zusammensetzungen enthaltend ein filmbildendes Polymer

Cosmetic compositions comprising a film-forming polymer

(84) Etats contractants désignés:
**AT BE CH DE ES FR GB IT LI NL SE**

(30) Priorité: **01.07.1997 FR 9708281**

(43) Date de publication de la demande:
**03.02.1999 Bulletin 1999/05**

(73) Titulaire: **L'OREAL**
**75008 Paris (FR)**

(72) Inventeurs:
- **Ramin, Roland**
**75014 Paris (FR)**
- **Gabin, Philippe**
**91440 Bures sur Yvette (FR)**
- **Frankfurt, Chris**
**NJ 08857 (US)**

(74) Mandataire: **Lhoste, Catherine**
**L'OREAL-DPI 6 rue Bertrand Sincholle**
**92585 Clichy Cédex (FR)**

(56) Documents cités:
**EP-A- 0 154 679**
**EP-A- 0 455 373**
**DD-A- 61 596**
**FR-A- 2 151 654**
**FR-A- 2 578 741**
**GB-A- 1 225 544**
**US-A- 5 100 944**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 894 490 B1

## Description

La présente invention concerne une nouvelle composition cosmétique. Plus particulièrement, l'invention concerne des compositions cosmétiques de maquillage et de soin comprenant au moins un polymère filmogène. Ces nouvelles compositions présentent des propriétés améliorées par rapport à l'art antérieur, en particulier en ce qui concerne la fluidité de la composition, la facilité d'application, l'homogénéité du film, la rapidité de séchage, la résistance du film à l'usure, la brillance, la résistance au jaunissement, la stabilité à la lumière et à la chaleur.

Les polymères filmogènes sont des constituants essentiels de nombreuses formes de cosmétiques, comme par exemple les vernis-à-ongles, les laques, les mascaras. Ils permettent de déposer le maquillage sous la forme d'un film à la surface de la peau, des cheveux et/ou des ongles. Habituellement on adjoint au polymère filmogène au moins un composé dit plastifiant dont le rôle est de conférer de la souplesse au film de maquillage. En effet, l'emploi d'un polymère filmogène seul donne un film rigide cassant, fragile, qui s'use rapidement. Toutefois, l'adjonction d'un plastifiant peut parfois entraîner d'autres inconvénients tels que le jaunissement, l'instabilité de la composition à la lumière et/ou à la chaleur, le manque de brillant, le manque de fluidité.

En effet, on connaît, en particulier par le document EP-154679, des compositions de vernis-à-ongles comprenant du N-éthyl-o,p-toluènesulfonamide (mélange des isomères ortho et para) comme agent plastifiant associé à un polymère filmogène. Toutefois, une telle association présente l'inconvénient de jaunir rapidement, d'être instable lorsqu'elle est exposée à la lumière et/ou à la chaleur.

Il est également connu d'utiliser un citrate, comme par exemple l'acétyl citrate de tributyle, comme agent plastifiant associé à un polymère filmogène. A cet effet, on pourra se reporter aux documents US-5,227,155 et FR-2 578 741. Toutefois, de telles compositions manquent de brillance, de fluidité, sont difficiles à appliquer.

Des compositions de colle comprenant à la fois un ester de l'acide citrique et un toluènesulfonamide sont connues par GB-1 225 544 et FR-2 151 654.

La présente invention vise à proposer une composition de maquillage présentant des propriétés améliorées par rapport à l'art antérieur.

C'est avec étonnement que la demanderesse a découvert que l'association d'un polymère filmogène avec un ester de l'acide citrique répondant à la formule (I) (ci-dessous) et d'un composé de formule (II) (ci-dessous) permettait de remédier aux inconvénients de l'art antérieur.

La présente invention a pour objet une composition cosmétique et/ou dermatologique comprenant, dans un support cosmétiquement et/ou dermatologiquement acceptable, :

(a) au moins un polymère filmogène ;

(b) au moins un composé choisi parmi les esters de l'acide citrique répondant à la formule (I) :

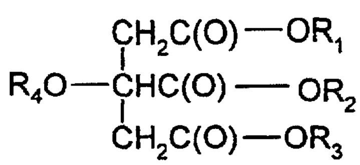

(I)

dans laquelle $R_1$, $R_2$, $R_3$, identiques ou différents représentent H ou une chaîne alkyle en $C_1$-$C_{30}$ linéaire, ramifiée ou cyclique, saturée ou insaturée, l'un au moins de $R_1$, $R_2$, $R_3$ étant distinct de l'atome d'hydrogène,

$R_4$ représente l'atome d'hydrogène ou un groupement $R'_4$-CO-, dans lequel $R'_4$ représente un groupement alkyle en $C_1$-$C_8$, linéaire, ramifié ou cyclique, saturé ou insaturé ;

(c) au moins un composé choisi parmi ceux répondant à la formule (II) :

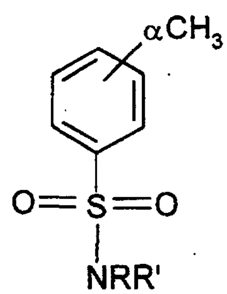

(II)

dans laquelle α représente la position ortho ou para du groupement méthyl, R et R', identiques ou différents représentent un atome d'hydrogène ou un groupement alkyle en $C_1$-$C_6$, linéaire, ramifié ou cyclique, saturé ou insaturé, à l'exclusion de :

i) la composition constituée de :

- 25 g d'acétate de polyvinyle de basse viscosité (produit "Rhodopass B" de la société Rhône Poulenc),
- 5 g d'acétylcitrate de tributyle,
- 30 g de mélange de o- et p-toluène-sulfonamides ("Santicizer 9" de la société Monsanto),
- 50 g d'acétone

ii) la composition X constituée d'une solution à 30 % de composition A dans le mélange de solvants de composition B :

Composition A :

- 10 parts de nitrocellulose
- 2,5 parts de phtalate de dicyclohexyle,
- 5,5 parts de citrate de tricyclohexyle
- 3 parts de N-cyclohexyl-p-toluène sulphonamide
- 4 parts de résine constituée d'un ester de glycérol modifié maléique et de collophane ayant un idice d'acide de 20 (mg KOH / g) et un point de fusion de 118-124 °C,
- 1 part de tartrate de di-n-butyle

Composition B :

- 25 parts d'acétate d'éthyle
- 25 parts d'acétate de n-butyle
- 10 parts de n-butanol
- 40 parts de toluène

iii) la composition Y constituée d'une solution de :

- 0,5 parts d'alcool polyvinylique,
- 0,5 parts de terpolymère de méthyl vinyl éther/anhydride maléique/allyl éther de nonyl phénol polyoxyéthylé,
- 22,5 parts d'eau

neutralisée à pH 7 avec une solution aqueuse à 10 % d'hydroxyde de sodium, et mélangée avec 75 parts de la composition X définie précédemment.

iv) la composition constituée de:

- 10 parts de nitrocellulose,
- 2,5 parts de phthalate de dicyclohexyle,
- 5,5 parts de citrate de tricyclohexyle
- 3 parts de N-cyclohexyl-p-toluène sulphonamide,
- 4 parts de résine produit de la condensation d'anhydride maléique et de rosine ayant un indice d'acide de 310 (mg KOH / g) et un point de fusion de 135-145 °C,
- 1 part de tartrate de di-n-butyle.

v) la composition constituée de:

- 10 parts de nitrocellulose
- 8 parts de citrate de tricyclohexyle,
- 3 parts de N-cyclohexyl-p-toluene sulphonamide
- 1 part de N-ethyl o/p-toluene sulphonamide,
- 8 parts de résine produit de la condensation d'anhydride maléique et de rosine ayant un indice d'acide de 310 (mg KOH / g) et un point de fusion de 135-145 °C.

L'invention a également pour objet une composition cosmétique et/ou dermatologique selon la revendication 2.

L'invention a aussi pour objet un procédé de maquillage ou de traitement cosmétique des cils, des sourcils, de la peau, des lèvres, des cheveux, des ongles consistant à appliquer sur ceux-ci une composition telle que définie précédemment.

La composition selon l'invention s'étale sur la peau, les muqueuses, les cils, les sourcils, les cheveux, la peau (paupières, joues), les lèvres ou les ongles avec une grande facilité, permet d'obtenir un film homogène, qui sèche rapidement et qui présente une résistance

**Revendications**

1. Composition cosmétique et/ou dermatologique **caractérisée par le fait qu'**elle comprend, dans un support cosmétiquement et/ou dermatologiquement acceptable :

   (a) au moins un polymère filmogène ;
   (b) au moins un composé choisi parmi les esters de l'acide citrique répondant à la formule (I) :

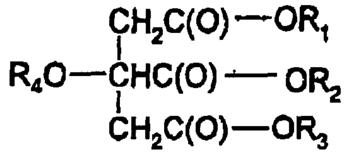

(I)

   dans laquelle $R_1$, $R_2$, $R_3$, identiques ou différents représentent H ou une chaîne alkyle en $C_1$-$C_{30}$ linéaire, ramifiée ou cyclique, saturée ou insaturée, l'un au moins de $R_1$, $R_2$, $R_3$ étant distinct de l'atome d'hydrogène,
   $R_4$ représente l'atome d'hydrogène ou un groupement $R'_4$-CO-, dans lequel $R'_4$ représente un groupement alkyle en $C_1$-$C_8$, linéaire, ramifié ou cyclique, saturé ou insaturé ;
   (c) au moins un composé choisi parmi ceux répondant à la formule (II) :

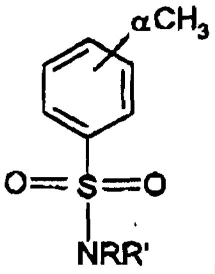

(II)

   dans laquelle α représente la position ortho ou para du groupement méthyl, R et R', identiques ou différents représentent un atome d'hydrogène ou un groupement alkyle en $C_1$-$C_6$, linéaire, ramifié ou cyclique, saturé ou insaturé;
   à l'exclusion de :

   i) la composition constituée de :

   - 25 g d'acétate de polyvinyle de basse viscosité (produit "Rhodopass B" de la société Rhône Poulenc),
   - 5 g d'acétylcitrate de tributyle,
   - 30 g de mélange de o- et p-toluène-sulfonamides ("Santicizer 9" de la société Monsanto),
   - 50 g d'acétone

   ii) la composition X constituée d'une solution à 30 % de composition A dans le mélange de solvants de composition B :

   Composition A :

- 10 parts de nitrocellulose
- 2,5 parts de phtalate de dicyclohexyle,
- 5,5 parts de citrate de tricyclohexyle
- 3 parts de N-cyclohexyl-p-toluène sulphonamide
- 4 parts de résine constituée d'un ester de glycérol modifié maléique et de cellophane ayant un indice d'acide de 20 (mg KOH / g) et un point de fusion de 118-124 °C,
- 1 part de tartrate de di-n-butyle

Composition B :

- 25 parts d'acétate d'éthyle
- 25 parts d'acétate de n-butyle
- 10 parts de n-butanol
- 40 parts de toluène

iii) la composition Y constituée :
d'une solution de

- 0,5 parts d'alcool polyvinylique.
- 0,5 parts de terpolymère de méthyl vinyl éther/anhydride maléique/allyl éther de nonyl phénol polyoxyéthylé,
- 22,5 parts d'eau

neutralisée à pH 7 avec une solution aqueuse à 10 % d'hydroxyde de sodium, et mélangée avec 75 parts de la composition X définie précédemment.

iv) la composition constituée de :

- 10 parts de nitrocellulose,
- 2.5 parts de phtalate de dicyclohexyle,
- 5,5 parts de citrate de tricyclohexyle,
- 3 parts de N-cyclohexyl-p-toluène sulphonamide,
- 4 parts de résine produit de la condensation d'anhydride maléique et de rosine ayant un indice d'acide de 310 (mg KOH/g) et un point de fusion de 135-145 °C,
- 1 part de tartrate de di-n-butyle.

v) la composition constituée de ;

- 10 parts de nitrocellulose,
- 8 parts de citrate de tricyclohexyle,
- 3 parts de N-cyclohexyl-p-toluene sulphonamide,
- 1 part de N-ethyl o/p-toluene sulphonamide,
- 8 parts de résine produit de la condensation d'anhydride maléique et de rosine ayant un indice d'acide de 310 (mg KOH/g) et un point de fusion de 135-145 °C.

**2.** Composition cosmétique et/ou dermatologique **caractérisée par le fait qu'**elle comprend, dans un support cosmétiquement et/ou dermatologiquement acceptable :

(a) au moins un polymère filmogène ;
(b) au moins un composé choisi parmi les esters de l'acide citrique répondant à la formule (I) :

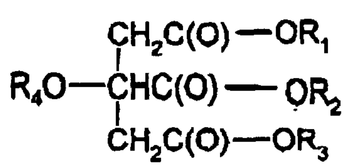

(I) .

dans laquelle $R_1$, $R_2$, $R_3$, identiques ou différents représentent H ou une chaîne alkyle en $C_1$-$C_{30}$ linéaire, ramifiée ou cyclique, saturée ou insaturée, l'un au moins de $R_1$, $R_2$, $R_3$ étant distinct de l'atome d'hydrogène,

$R_4$ représente l'atome d'hydrogène ou un groupement $R'_4$-CO-, dans lequel $R'_4$ représente un groupement alkyle en $C_1$-$C_8$, linéaire, ramifié ou cyclique, saturé ou insaturé ;

(c) au moins un composé choisi parmi ceux répondant à la formule (II) :

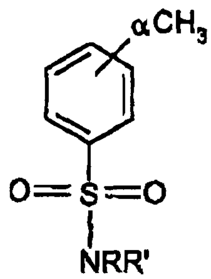

(II)

dans laquelle α représente la position ortho ou para du groupement méthyl, R et R', identiques ou différents représentent un atome d'hydrogène ou un groupement alkyle en $C_1$-$C_8$, linéaire, ramifié ou cyclique, saturé ou insaturé, et

(d) au moins un ingrédient choisi dans le groupe formé par :

- les agents de coloration choisi parmi les pigments minéraux, les pigments organiques, les laques, les pigments nacrés ;
- les charges choisies parmi le talc, les micas, l'amidon, la silice, le kaolin, les poudres de Nylon® et de polyéthylène, le Téflon® , le nitrure de bore, les microbilles de résine de slicone, le carbonate de calcium précipité, le carbonate ou l'hydrocarbonate de magnésium, les savons métalliques dérivés d'acides organiques carboxyliques ayant de 8 à 22 atomes de carbone ;
- les conservateurs, les parfums.

**3.** Composition selon la revendication 1 ou 2, **caractérisée par le fait que** le composé de formule (I) est choisi parmi le citrate de triéthyl, le citrate de tributyle, l'acétylcitrate de triéthyl, l'acétylcitrate de triéthyl-2 hexyle.

**4.** Composition selon l'une quelconque des revendications 1 à 3, **caractérisée par le fait que** le composé de formule (II) est le N-éthyl-o,p-toluènesulfonamide.

**5.** Composition selon l'une quelconque des revendications précédentes, **caractérisée par le fait que** le polymère filmogène (a), le composé (b) de formule (I) et le composé (c) de formule (II) sont présents dans des quantités qui vérifient la relation :

$$1 <(a)/[(b)+(c)] < 4$$

**6.** Composition selon la revendication 5, **caractérisée par le fait que** le polymère filmogène (a), le composé (b) de formule (I) et le composé (c) de formule (II) sont présents dans des quantités qul vérifient la relation :

$$1,5 <(a)/[(b)+(c)] < 2,5$$

**7.** Composition selon rune quelconque des revendications 1 à 6, **caractérisée par le fait que** le composé (b) de formule (I) et le composé (c) de formule (II) sont présents dans des quantités qui vérifient la relation :

$$0,3 < (b)/(c) < 3$$

**8.** Composition selon la revendication 7, **caractérisée par le fait que** le composé (b) de formule (I) et le composé (c) de formule (II) sont présents dans des quantités qui vérifient la relation :

$$0,8 < (b)/(c) < 1,5$$

**9.** Composition selon l'une quelconque des revendications précédentes, **caractérisée par le fait que** le polymère filmogène est choisi parmi les hydrolysats de kératine, les kératines sulfoniques ; les dérivés de chitine ou de chitosane anioniques, cationiques, amphotères ou non-ioniques ; l'hydroxyéthylcellulose, l'hydroxypropyl-cellulose, la méthylcellulose, l'éthylhydroxyéthylcellulose, la carboxyméthylcellulose, les polymères acryliques, les résines styrénique, acrylate-styrénique, les polyvinylpyrrolidones, les copolymères de l'éther méthylvinylique et de l'anhydride malique, le copolymère de l'acétate de vinyle et de l'acide crotonique ; les polyesters, les polyamides, les résines époxyesters, les polyuréthannes, les polyurées, les polymères d'origine naturelle, les résines du type aryl-sulfonamide formaldéhyde ou aryl-sulfonamide époxy.

**10.** Composition selon l'une quelconque des revendications précédentes, **caractérisée par le fait que** le polymère filmogène est choisi parmi les nitrocelluloses du type « RS » ou « SS ».

**11.** Composition selon l'une quelconque des revendications précédentes, **caractérisée par le fait qu'**elle est destinée au maquillage des cils et/ou des sourcils et/ou des cheveux et/ou de la peau et/ou des lèvres et/ou des ongles.

**12.** Composition selon rune quelconque des revendications précédentes destinée à être "appliquée sur les cils, les sourcils, la peau, les lèvres et/ou les cheveux, **caractérisée par le fait qu'**elle comprend une quantité de polymère filmogène allant de 0,1 à 25 % en poids par rapport au poids total de la composition, avantageusement, de 1 à 10 %.

**13.** Composition selon l'une quelconque des revendications 1 à 11 destinée à être appliquée sur les ongles, **caractérisée par le fait qu'**elle comprend une quantité de matière filmogène allant de 2 à 40 %, prélérentiellement de 5 à 25% en poids par rapport au poids total de la composition.

**14.** Composition selon l'une quelconque des revendications 1 et 3 à 13, **caractérisée par le fait qu'**elle comprend un ingrédient choisi parmi les charges, les adoucissants, les conservateurs, les séquestrants, les parfums, les épaississants, les agents de cohésion, les agents alcalinisants ou acidifiants, les hydratants, les actifs hydrosolubles.

**15.** Composition selon l'une quelconque des revendications 1 à 13, caractsrisée par le fait qu'elle comprend un ingrédient choisi parmi les adoucissants, les agents de cohésion, les agents alcalinisants ou acidifiants, les hydratants, les actifs hydrosolubles.

**16.** Composition selon l'une quelconque des revendications précédentes, **caractérisée par le fait qu'**elle comprend au moins un agent de coloration en quantité allant de 0,01 à 60 % en poids par rapport au poids total de la composition, préférentiellement de 0,05 à 30 %, encore plus préférentiellement de 0,5 à 5%.

**17.** Composition selon l'une quelconque des revendications précédentes, **caractérisée par le fait qu'**elle se présente sous forme d'une émulsion huile-dans-l'eau ou eau-dans-l'huile, d'une solution en milieu solvant organique ou aqueux, d'une suspension en milieu solvant organique ou aqueux, sous forme de gel ou de mousse.

**18.** Composition selon l'une quelconque des revendications 1 à 11, et 13 à 17 précédentes, **caractérisée par le fait qu'**elle se présente sous forme d'un vemis à ongles ou d'une base de soin pour ongles anhydre.

**19.** Composition selon l'une quelconque des revendications 1 à 11, et 13 à 17 précédentes, **caractérisée par le fait qu'**elle se présente sous forme d'un vernis à ongles ou d'une base de soin pour ongles aqueux.

**20.** Composition selon l'une quelconque des revendications précédentes, **caractérisée par le fait qu'**elle contient une quantité totale d'agent plastifiant allant de 2 à 20 %, préférentiellement de 5 à 15% en poids par rapport au poids total de la composition.

**21.** Procédé de maquillage des cils, des sourcils, de la peau, des lèvres, des cheveux **caractérisé par le fait que** l'on applique sur ceux-ci une composition selon lune quelconque des revendications 1 à 11 et 13 à 15.

**22.** Procédé de traitement cosmétique des cils, des sourcils, de la peau, des lèvres, des cheveux **caractérisé par le fait que** l'on applique sur ceux-ci une composition selon l'une quelconque des revendications 1 à 11 et 13 à 15.

**23.** Procédé de maquillage des ongles **caractérisé par le fait que** l'on applique sur ceux-ci une composition selon l'une quelconque des revendications 1 à 9 et 12 à 18.

**24.** Procédé de traitement cosmétique des ongles **caractérisé par le fait que** l'on applique sur les ongles une composition selon l'une quelconque des revendications 1 à 9 et 12 à 18.

**25.** Utilisation d'un composé de formule (I) et d'un composé de formule (II) tels que définis dans rune des revendications 1 à 4, dans une composition cosmétique et/ou dermatologique comprenant, dans un support cosmétiquement acceptable, un polymère filmogène, pour obtenir une composition résistante au jaunissement et/ou stable à la lumière et/ou stable à la chaleur.

**26.** Utilisation d'un composé de formule (I) et d'un composé de formule (II) tels que définis dans l'une des revendications 1 à 4, dans une composition cosmétique et/ou dermatologique comprenant, dans un support cosmétiquement acceptable, un polymère filmogène, pour obtenir un'film brillant, à l'exclusion des méthodes de traitement thérapeutique du corps humain ou animal.

**27.** Utilisation selon l'une des revendications 25 ou 26, **caractérisée par le fait que** le polymère filmogène est choisi parmi les nitrocelluloses du type « RS » ou « SS ».

**28.** Utilisation d'une composition selon l'une quelconque des revendications 1 à 20 pour obtenir un film brillant, à l'exclusion des méthodes de traitement thérapeutique du corps humain ou animal.

**Claims**

**1.** Cosmetic and/or dermatological composition, **characterized in that** it comprises, in a cosmetically and/or dermatologically acceptable vehicle:

(a) at least one film-forming polymer;
(b) at least one compound chosen from citric acid esters corresponding to the formula (I):

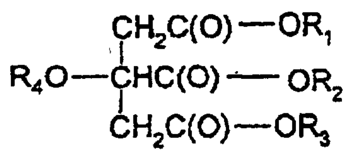

(I)

in which $R_1$, $R_2$ and $R_3$, which are identical or different, represent H or a saturated or unsaturated and linear, branched or cyclic $C_1$-$C_{30}$ alkyl chain, at least one of $R_1$, $R_2$ and $R_3$ being other than a hydrogen atom,
$R_4$ represents a hydrogen atom or an $R'_4$-CO- group, in which $R'_4$ represents a saturated or unsaturated and linear, branched or cyclic $C_1$-$C_8$ alkyl group;
(c) at least one compound chosen from those corresponding to the formula (II) :

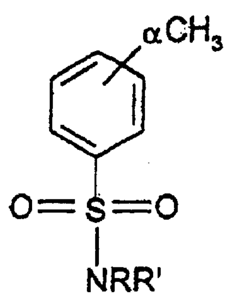

(II)

in which α represents the ortho or para position of the methyl group and R and R', which are identical or different, represent a hydrogen atom or a saturated or unsaturated and linear, branched or cyclic $C_1$-$C_6$ alkyl group; with the exception of:

i) the composition composed of:

- 25 g of low viscosity poly (vinyl acetate) ("Rhodopass B" product from Rhône-Poulenc),
- 5 g of tributyl acetylcitrate,
- 30 g of mixture of o- and p-toluenesulphonamides ("Santicizer . 9" from Monsanto),
- 50 g of acetone,

ii) the composition X composed of a 30% solution of composition A in the mixture of solvents of composition B:

Composition A:

- 10 parts of nitrocellulose,
- 2.5 parts of dicyclohexyl phthalate,
- 5.5 parts of tricyclohexyl citrate,
- 3 parts of N-cyclohexyl-p-toluenesulphonamide,
- 4 parts of resin composed of an ester of glycerol, which is maleic modified, and of rosin having an acid number of 20 (mg KOH/g) and a melting point of 118-124°C,
- 1 part of di(n-butyl) tartrate,

Composition B:

- 25 parts of ethyl acetate,
- 25 parts of n-butyl acetate,
- 10 parts of n-butanol,
- 40 parts of toluene,

iii) the solution Y composed:
of a solution of

- 0.5 part of poly(vinyl alcohol),
- 0.5 part of methyl vinyl ether/maleic anhydride/polyoxyethylenated nonylphenol allyl ether terpolymer,
- 22.5 parts of water,

neutralized to pH 7 with a 10% aqueous sodium hydroxide solution, and mixed with 75 parts of the composition X defined above,

iv) the composition composed of:

- 10 parts of nitrocellulose,
- 2.5 parts of dicyclohexyl phthalate,
- 5.5 parts of tricyclohexyl citrate,
- 3 parts of N-cyclohexyl-p-toluene sulphonamide,
- 4 parts of resin produced from the condensation of maleic anhydride and of rosin, having an acid number of 310 (mg KOH/g) and a melting point of 135-145°C,
- 1 part of di(n-butyl) tartrate,

v) the composition composed of:

- 10 parts of nitrocellulose,
- 8 parts of tricyclohexyl citrate,
- 3 parts of N-cyclohexyl-p-toluenesulphonamide,
- 1 part of N-ethyl-o/p-toluenesulphonamide,
- 8 parts of resin produced from the condensation of maleic anhydride and of rosin, having an acid number of 310 (mg KOH/g) and a melting point of 135-145°C.

**2.** Cosmetic and/or dermatological composition, **characterized in that** it comprises, in a cosmetically and/or dermatologically acceptable vehicle:

(a) at least one film-forming polymer;
(b) at least one compound chosen from citric acid esters corresponding to the formula (I):

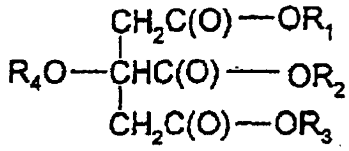 (I)

in which $R_1$, $R_2$ and $R_3$, which are identical or different, represent H or a saturated or unsaturated and linear, branched or cyclic $C_1$-$C_{30}$ alkyl chain, at least one of $R_1$, $R_2$ and $R_3$ being other than a hydrogen atom, $R_4$ represents a hydrogen atom or an $R'_4$-CO- group, in which $R'_4$ represents a saturated or unsaturated and linear, branched or cyclic $C_1$-$C_8$ alkyl group;
(c) at least one compound chosen from those corresponding to the formula (II) :

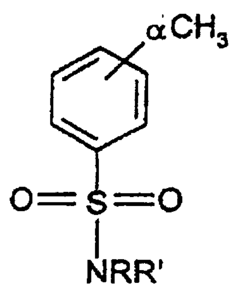 (II)

in which $\alpha$ represents the ortho or para position of the methyl group and R and R', which are identical or different, represent a hydrogen atom or a saturated or unsaturated and linear, branched or cyclic $C_1$-$C_6$ alkyl group, and
(d) at least one ingredient chosen from the group formed by:

- colouring agents chosen from inorganic pigments, organic pigments, lakes or pearlescent pigments;
- fillers chosen from talc, micas, starch, silica, kaolin, Nylon® and polyethylene powders, Teflon®, boron nitride, silicone resin microbeads, precipitated calcium carbonate, magnesium carbonate or basic magnesium carbonate, or metal soaps derived from organic carboxylic acids having from 8 to 22 carbon atoms;
- preservatives and fragrances.

**3.** Composition according to Claim 1 or 2, **characterized in that** the compound of formula (I) is chosen from triethyl citrate, tributyl citrate, triethyl acetylcitrate or tri(2-ethylhexyl) acetylcitrate.

**4.** Composition according to any one of Claims 1 to 3, **characterized in that** the compound of formula (II) is N-ethyl-o,p-toluenesulphonamide.

**5.** Composition according to any one of the preceding claims, **characterized in that** the film-forming polymer (a), the compound (b) of formula (I) and the compound (c) of formula (II) are present in amounts which obey the relationship:

$$1 < (a)/[(b)+(c)] < 4$$

**6.** Composition according to Claim 5, **characterized in that** the film-forming polymer (a), the compound (b) of formula (I) and the compound (c) of formula (II) are present in amounts which obey the relationship:

$$1.5 < (a)/[(b)+(c)] < 2.5$$

**7.** Composition according to any one of Claims 1 to 6, **characterized in that** the compound (b) of formula (I) and the compound (c) of formula (II) are present in amounts which obey the relationship:

$$0.3 < (b)/(c) < 3$$

**8.** Composition according to Claim 7, **characterized in that** the compound (b) of formula (I) and the compound (c) of formula (II) are present in amounts which obey the relationship:

$$0.8 < (b)/(c) < 1.5$$

**9.** Composition according to any one of the preceding claims, **characterized in that** the film-forming polymer is chosen from keratin hydrolysates, sulphonic keratins, anionic, cationic, amphoteric or nonionic chitosan or chitin derivatives, hydroxyethylcellulose, hydroxypropylcellulose, methylcellulose, ethylhydroxyethylcellulose, carboxymethylcellulose, acrylic polymers, styrene or acrylate-styrene resins, polyvinylpyrrolidones, copolymers of methyl vinyl ether and of malic anhydride, the copolymer of vinyl acetate and of crotonic acid, polyesters, polyamides, epoxy ester resins, polyurethanes, polyureas, polymers of natural origin, or resins of the arylsulphonamideformaldehyde or arylsulphonamide-epoxy type.

**10.** Composition according to any one of the preceding claims, **characterized in that** the film-forming polymer is chosen from nitrocelluloses of the "RS" or "SS" type.

**11.** Composition according to any one of the preceding claims, **characterized in that** it is intended for making up the eyelashes and/or eyebrows and/or hair and/or skin and/or lips and/or nails.

**12.** Composition according to any one of the preceding claims intended to be applied to the eyelashes, eyebrows, skin, lips and/or hair, **characterized in that** it comprises an amount of film-forming polymer ranging from 0.1 to 25% by weight with respect to the total weight of the composition, advantageously from 1 to 10%.

**13.** Composition according to any one of Claims 1 to 11 intended to be applied to the nails, **characterized in that** it comprises an amount of film-forming material ranging from 2 to 40%, preferably from 5 to 25%, by weight with respect to the total weight of the composition.

**14.** Composition according to any one of Claims 1 and 3 to 13, **characterized in that** it comprises an ingredient chosen from fillers, softeners, preservatives, sequestering agents, fragrances, thickeners, cohesion agents, basifying or acidifying agents, moisturizers or water-soluble active principles.

**15.** Composition according to any one of Claims 1 to 13, **characterized in that** it comprises an ingredient chosen from softeners, cohesion agents, basifying or acidifying agents, moisturizers or water-soluble active principles.

**16.** Composition according to any one of the preceding claims, **characterized in that** it comprises at least one colouring agent in an amount ranging from 0.01 to 60% by weight with respect to the total weight of the composition, preferably from 0.05 to 30%, more preferably still from 0.5 to 5%.

**17.** Composition according to any one of the preceding claims, **characterized in that** it is provided in the form of an oil-in-water or water-in-oil emulsion, of a solution in an aqueous or organic solvent medium or of a suspension in an aqueous or organic solvent medium or in the form of a gel or foam.

**18.** Composition according to any one of the preceding Claims 1 to 11 and 13 to 17, **characterized in that** it is provided in the form of an anhydrous nail varnish or of an anhydrous nail-care base.

**19.** Composition according to any one of the preceding Claims 1 to 11 and 13 to 17, **characterized in that** it is provided in the form of an aqueous nail varnish or of an aqueous nail-care base.

**20.** Composition according to any one of the preceding claims, **characterized in that** it comprises a total amount of plasticizing agent ranging from 2 to 20%, preferably from 5 to 15%, by weight with respect to the total weight of the composition.

**21.** Process for making up the eyelashes, eyebrows, skin, lips or hair, **characterized in that** a composition according to any one of Claims 1 to 11 and 13 to 15 is applied to these.

**22.** Process for the cosmetic treatment of the eyelashes, eyebrows, skin, lips or hair, **characterized in that** a composition according to any one of Claims 1 to 11 and 13 to 15 is applied to these.

**23.** Process for making up the nails, **characterized in that** a composition according to any one of Claims 1 to 9 and 12 to 18 is applied to these.

**24.** Process for the cosmetic treatment of the nails, **characterized in that** a composition according to any one of Claims 1 to 9 and 12 to 18 is applied to the nails.

**25.** Use of a compound of formula (I) and of a compound of formula (II) as defined in one of Claims 1 to 4, in a cosmetic and/or dermatological composition comprising, in a cosmetically acceptable vehicle, a film-forming polymer, to produce a composition which withstands yellowing and/or is stable to light and/or is stable to heat.

**26.** Use of a compound of formula (I) and of a compound of formula (II) as defined in one of Claims 1 to 4, in a cosmetic and/or dermatological composition comprising, in a cosmetically acceptable vehicle, a film-forming polymer, to produce a glossy film, with the exception of the methods for the therapeutic treatment of the human or animal body.

**27.** Use according to either of Claims 25 and 26, **characterized in that** the film-forming polymer is chosen from nitrocelluloses of the "RS" or "SS" type.

**28.** Use of a composition according to any one of Claims 1 to 20 to produce a glossy film, with the exception of the methods for the therapeutic treatment of the human or animal body.

**Patentansprüche**

**1.** Kosmetische und/oder dermatologische Zusammensetzung, **dadurch gekennzeichnet, daß** sie in einem kosmetisch und/oder dermatologisch akzeptablen Träger enthält:

(a) mindestens ein filmbildendes Polymer;

(b) mindestens eine Verbindung, die unter den Citronensäureestern der Formel (I) ausgewählt ist:

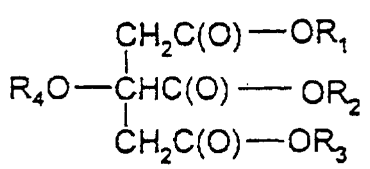

(I),

worin die Gruppen $R_1$, $R_2$ und $R_3$, die identisch oder voneinander verschieden sind, unter H oder einer geradkettigen, verzweigten oder cyclischen, gesättigten oder ungesättigten $C_{1-30}$-Alkylgruppe ausgewählt sind, wobei mindestens eine der Gruppen $R_1$, $R_2$ und $R_3$ von Wasserstoff verschieden ist, und die Gruppe $R_4$ Wasserstoff oder eine Gruppe $R'_4$-CO- bedeutet, wobei $R'_4$ eine geradkettige, verzweigte oder cyclische, gesättigte oder ungesättigte $C_1$-$_8$-Alkylgruppe ist;

(c) mindestens eine Verbindung, die unter den Verbindungen der Formel (II) ausgewählt ist:

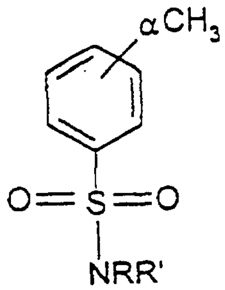

(II),

worin α die ortho- oder para-Stellung der Methylgruppe bedeutet und die Gruppen R und R', die identisch oder voneinander verschieden sind, ein Wasserstoffatom oder eine geradkettige, verzweigte oder cyclische, gesättigte oder ungesättigte $C_{1-6}$-Alkylgruppe bedeuten;

wobei ausgenommen sind:

i) die Zusammensetzung, die besteht aus:

- 25 g Polyvinylacetat niedriger Viskosität (Produkt "Rhodopass B" der Firma Rhône Poulenc),
- 5 g Tributylacetylcitrat,
- 30 g Gemisch von o- und p-Toluolsulfonamid ("Santicizer 9" der Firma Monsanto),
- 50 g Aceton;

ii) die Zusammensetzung X, die aus einer Lösung von 30 % Zusammensetzung A in dem Lösungsmittelgemisch der Zusammensetzung B besteht:

Zusammensetzung A:

- 10 Teile Nitrocellulose,
- 2,5 Teile Dicyclohexylphthalat,
- 5,5 Teile Tricyclohexylcitrat,
- 3 Teile N-Cyclohexyl-p-toluolsulfonamid,
- 4 Teile Harz, das aus einem mit Maleinsäure und Kolophonium modifizierten Glycerinester besteht und einen Säureindex von 20 (mg KOH/g) und einen Schmelzpunkt von 118 - 124 °C aufweist,
- 1 Teil Di-n-butyltartrat;

Zusammensetzung B:

- 25 Teile Ethylacetat,
- 25 Teile n-Butylacetat,
- 10 Teile n-Butanol,
- 40 Teile Toluol;

iii) die Zusammensetzung Y, die besteht aus:
einer Lösung von

- 0,5 Teilen Polyvinylalkohol,
- 0,5 Teilen Terpolymer Methylvinylether/Maleinsäureanhydrid/polyethoxylierter Nonylphenolallylether,
- 22,5 Teilen Wasser,

mit einer wäßrigen Lösung von 10 % Natriumhydroxid auf pH 7 eingestellt, im Gemisch mit 75 Teilen der oben definierten Zusammensetzung X;
iv) die Zusammensetzung, die besteht aus:

- 10 Teilen Nitrocellulose,
- 2,5 Teilen Dicyclohexylphthalat,
- 5,5 Teilen Tricyclohexylcitrat,
- 3 Teilen N-Cyclohexyl-p-toluolsulfonamid,
- 4 Teilen Harz, das das Kondensationsprodukt von Maleinsäureanhydrid und Rosin ist und einen Säureindex von 310 (mg KOH/g) und einen Schmelzpunkt von 135 - 145 °C aufweist,
- 1 Teil Di-n-butyltartrat;

v) die Zusammensetzung, die besteht aus:

- 10 Teilen Nitrocellulose,
- 8 Teilen Tricyclohexylcitrat,
- 3 Teilen N-Cyclohexyl-p-toluolsulfonamid,
- 1 Teil N-Ethyl-o/p-toluolsulfonamid,
- 8 Teilen Harz, das das Kondensationsprodukt von Maleinsäureanhydrid und Rosin ist und einen Säureindex von 310 (mg KOH/g) und einen Schmelzpunkt von 135 - 145 °C aufweist.

**2.** Kosmetische und/oder dermatologische Zusammensetzung, **dadurch gekennzeichnet, daß** sie in einem kosmetisch und/oder dermatologisch akzeptablen Träger enthält:

(a) mindestens ein filmbildendes Polymer;

(b) mindestens eine Verbindung, die unter den Citronensäureestern der Formel (I) ausgewählt ist:

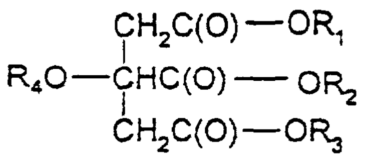

(I),

worin die Gruppen $R_1$, $R_2$ und $R_3$, die identisch oder voneinander verschieden sind, unter H oder einer geradkettigen, verzweigten oder cyclischen, gesättigten oder ungesättigten $C_{1-30}$-Alkylgruppe ausgewählt sind, wobei mindestens eine der Gruppen $R_1$, $R_2$ und $R_3$ von Wasserstoff verschieden ist, und die Gruppe $R_4$ Wasserstoff oder eine Gruppe $R'_4$-CO- bedeutet, wobei $R'_4$ eine geradkettige, verzweigte oder cyclische, gesättigte oder ungesättigte $C_{1-8}$-Alkylgruppe bedeutet;

(c) mindestens eine Verbindung, die unter den Verbindungen der Formel (II) ausgewählt ist:

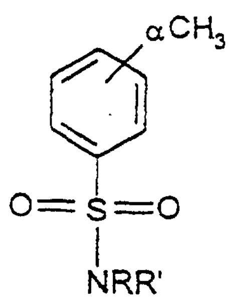

(II),

worin $\alpha$ die ortho- oder para-Stellung der Methylgruppe bedeutet und die Gruppen R und R', die identisch oder voneinander verschieden sind, ein Wasserstoffatom oder eine geradkettige, verzweigte oder cyclische, gesättigte oder ungesättigte $C_{1-6}$-Alkylgruppe bedeuten; und

(d) mindestens einen Bestandteil, der ausgewählt ist unter:

- den Farbmitteln, die unter den anorganischen Pigmenten, organischen Pigmenten, Lacken und Perlglanzpigmenten ausgewählt sind;
- Füllstoffen, die unter Talk, Glimmern, Stärke, Kieselsäure, Kaolin, Pulvern von Nylon® und Polyethylen, Teflon® , Bornitrid, Siliconharzmikrokugeln, gefälltem Calciumcarbonat, Magnesiumcarbonat oder Magnesiumhydrogencarbonat und Metallseifen, die von organischen Carbonsäuren mit 8 bis 22 Kohlenstoffatomen abgeleitet sind, ausgewählt sind; und
- Konservierungsmitteln und Parfums.

**3.** Zusammensetzung nach Anspruch 1 oder 2, **dadurch gekennzeichnet, daß** die Verbindung der Formel (I) unter Triethylcitrat, Tributylcitrat, Triethylacetylcitrat und 2-Triethylhexylacetylcitrat ausgewählt ist.

**4.** Zusammensetzung nach einem der Ansprüche 1 bis 3, **dadurch gekennzeichnet, daß** die Verbindung der Formel (II) das N-Ethyl-o,p-toluolsulfonamid ist.

**5.** Zusammensetzung nach einem der vorhergehenden Ansprüche, **dadurch gekennzeichnet, daß** das filmbildende Polymer (a), die Verbindung (b) der Formel (I) und die Verbindung (c) der Formel (II) in solchen Mengen vorliegen, daß die folgende Beziehung erfüllt wird:

$$1 < (a)/[(b) + (c)] < 4.$$

**6.** Zusammensetzung nach Anspruch 5, **dadurch gekennzeichnet, daß** das filmbildende Polymer (a), die Verbindung (b) der Formel (I) und die Verbindung (c) der Formel (II) in solchen Mengen vorliegen, daß die Beziehung erfüllt wird:

$$1{,}5 < (a)/[(b) + (c)] < 2{,}5.$$

**7.** Zusammensetzung nach einem der Ansprüche 1 bis 6, **dadurch gekennzeichnet, daß** die Verbindung (b) der Formel (I) und die Verbindung (c) der Formel (II) in solchen Mengen vorliegen, daß die Beziehung erfüllt wird:

$$0{,}3 < (b)/(c) < 3.$$

**8.** Zusammensetzung nach Anspruch 7, **dadurch gekennzeichnet, daß** die Verbindung (b) der Formel (I) und die Verbindung (c) der Formel (II) in solchen Mengen vorliegen, daß die Beziehung erfüllt wird:

$$0{,}8 < (b)/(c) < 1{,}5.$$

**9.** Zusammensetzung nach einem der vorhergehenden Ansprüche, **dadurch gekennzeichnet, daß** das filmbildende Polymer ausgewählt ist unter: Keratinhydrolysaten, Sulfokeratinen; anionischen, kationischen, amphoteren oder nichtionischen Chitinderivaten oder Chitosanderivaten; Hydroxyethylcellulose, Hydroxypropylcellulose, Methylcellulose, Ethylhydroxyethylcellulose, Carboxymethylcellulose, Acrylpolymeren, Styrolharzen, Acrylat-Styrolverbindungen, Polyvinylpyrrolidonen, Copolymeren von Methylvinylether und Maleinsäureanhydrid, dem Vinylacetat/Crotonsäure-Copolyrner; Polyestern, Polyamiden, Epoxyesterharzen, Polyurethanen, Polyharnstoffen, Polymeren natürlicher Herkunft und Harzen vom Typ Arylsulfonamid/Formaldehyd oder Arylsulfonamid/Epoxy.

**10.** Zusammensetzung nach einem der vorhergehenden Ansprüche, **dadurch gekennzeichnet, daß** das filmbildende Polymer unter den Nitrocellulosen vom Typ "RS" oder "SS" ausgewählt ist.

**11.** Zusammensetzung nach einem der vorhergehenden Ansprüche, **dadurch gekennzeichnet, daß** sie zum Schminken der Wimpern und/oder der Augenbrauen und/oder der Haare und/oder der Haut und/oder der Lippen und/oder der Nägel vorgesehen ist.

**12.** Zusammensetzung nach einem der vorhergehenden Ansprüche, die auf die Wimpern, Augenbrauen, Haut, Lippen und/oder Haare aufgetragen werden soll, **dadurch gekennzeichnet, daß** sie das filmbildende Polymer in einer Menge von 0,1 bis 25 Gew.-%, bezogen auf das Gesamtgewicht der Zusammensetzung, und vorteilhaft von 1 bis

10 % enthält.

**13.** Zusammensetzung nach einem der Ansprüche 1 bis 11, die auf die Nägel aufgetragen werden soll, **dadurch gekennzeichnet, daß** sie das filmbildende Material in einer Menge von 2 bis 40 % und vorzugsweise 5 bis 25 Gew.-%, bezogen auf das Gesamtgewicht der Zusammensetzung, enthält.

**14.** Zusammensetzung nach einem der Ansprüche 1 und 3 bis 13, **dadurch gekennzeichnet, daß** sie einen Bestandteil enthält, der unter den Füllstoffen, reizlindernden Mitteln, Konservierungsmitteln, Maskierungsmitteln, Parfums, Verdickungsmitteln, Kohäsionsmitteln, Alkalisierungs- oder Ansäuerungsmitteln, Hydratisierungsmitteln und wasserlöslichen Wirkstoffen ausgewählt ist.

**15.** Zusammensetzung nach einem der Ansprüche 1 bis 13, **dadurch gekennzeichnet, daß** sie einen Bestandteil enthält, der unter den reizlindernden Mitteln, Kohäsionsmitteln, Alkalisierungs- oder Ansäuerungsmitteln, Hydratisierungsmitteln und wasserlöslichen Wirkstoffen ausgewählt ist.

**16.** Zusammensetzung nach einem der vorhergehenden Ansprüche, **dadurch gekennzeichnet, daß** sie mindestens ein Farbmittel in einer Menge von 0,01 bis 60 Gew.-%, bezogen auf das Gesamtgewicht der Zusammensetzung, vorzugsweise von 0,05 bis 30 % und noch bevorzugter 0,5 bis 5 % enthält.

**17.** Zusammensetzung nach einem der vorhergehenden Ansprüche, **dadurch gekennzeichnet, daß** sie in Form einer Öl-in-Wasser-Emulsion, Wasser-in-Öl-Emulsion, Lösung in einem organischen oder wäßrigen Lösemittelmedium, Suspension in einem organischen oder wäßrigen Lösemittelmedium oder in Form von Gel oder Schaum vorliegt.

**18.** Zusammensetzung nach einem der Ansprüche 1 bis 11 und 13 bis 17, **dadurch gekennzeichnet, daß** sie in Form von wasserfreien Nagellacken oder Pflege-Nagelgrundierungen vorliegt.

**19.** Zusammensetzung nach einem der Ansprüche 1 bis 11 und 13 bis 17, **dadurch gekennzeichnet, daß** sie als wäßriger Nagellack oder als wäßrige Pflege-Nagelgrundierung vorliegt.

**20.** Zusammensetzung nach einem der vorhergehenden Ansprüche, **dadurch gekennzeichnet, daß** sie den Weichmacher in einer Gesamtmenge von 2 bis 20 % und vorzugsweise 5 bis 15 Gew.-%, bezogen auf das Gesamtgewicht der Zusammensetzung, enthält.

**21.** Verfahren zum Schminken der Wimpern, Augenbrauen, Haut, Lippen und Haare, **dadurch gekennzeichnet, daß** auf diese eine Zusammensetzung nach einem der Ansprüche 1 bis 11 und 13 bis 15 aufgetragen wird.

**22.** Verfahren zur kosmetischen Behandlung der Wimpern, Augenbrauen, Haut, Lippen und Haare, **dadurch gekennzeichnet, daß** auf diese eine Zusammensetzung nach einem der Ansprüche 1 bis 11 und 13 bis 15 aufgetragen wird.

**23.** Verfahren zum Schminken der Nägel, **dadurch gekennzeichnet, daß** auf diese eine Zusammensetzung nach einem der Ansprüche 1 bis 9 und 12 bis 18 aufgetragen wird.

**24.** Verfahren zur kosmetischen Behandlung der Nägel, **dadurch gekennzeichnet, daß** auf die Nägel eine Zusammensetzung nach einem der Ansprüche 1 bis 9 und 12 bis 18 aufgetragen wird.

**25.** Verwendung einer Verbindung der Formel (I) und einer Verbindung (II) nach einem der Ansprüche 1 bis 4 in einer kosmetischen und/oder dermatologischen Zusammensetzung, die in einem kosmetisch akzeptablen Träger ein filmbildendes Polymer enthält, um eine Zusammensetzung herzustellen, die gegenüber Gelbfärbung beständig und/oder lichtstabil und/oder wärmestabil ist.

**26.** Verwendung einer Verbindung der Formel (I) und einer Verbindung der Formel (II) nach einem der Ansprüche 1 bis 4 in einer kosmetischen und/oder dermatologischen Zusammensetzung, die in einem kosmetisch akzeptablen Träger ein filmbildendes Polymer enthält, um einen glänzenden Film herzustellen, wobei therapeutische Verfahren zur Behandlung des menschlichen oder tierischen Körpers ausgenommen sind.

**27.** Verwendung nach einem der Ansprüche 25 oder 26, **dadurch gekennzeichnet, daß** das filmbildende Polymer unter den Nitrocellulosen vom Typ "RS" oder "SS" ausgewählt ist.

**28.** Verwendung einer Zusammensetzung nach einem der Ansprüche 1 bis 20 um einen glänzenden Film herzustellen, wobei Verfahren zur therapeutischen Behandlung des menschlichen oder tierischen Körpers ausgenommen sind.